# EUROPEAN PATENT APPLICATION

(11) **EP 4 763 087 A1**
(43) Date of publication of application: **24.06.2026**
(21) Application number: 24221574.7
(22) Date of filing: 19.12.2024
(51) Int. Cl.: A61B 5/349, A61B 5/00

(54) **VITAL SIGN MONITORING SYSTEM AND METHOD**

(71) Applicant: Novosense AB, 223 63 Lund (SE)
(72) Inventor: TILLY, Jonas, 223 63 Lund (SE); SEBELIUS, Fredrik, 223 63 Lund (SE)
(74) Representative: AWA Sweden AB

(57) **Abstract**

A vital sign monitoring system, comprising at least two vital sign sensors being configured to measure a same first vital sign parameter, a data processing unit configured to: receive data pertaining to said vital sign parameter from each one of said at least two vital sign sensors; analyze said data based on a criteria, and generate an output based on said analysis.

## Description

### Field of the invention

The invention relates to a vital sign monitoring system, and a method for monitoring vital signs.

### Background art

Vital sign monitoring systems play a crucial role in diagnosing, monitoring, and preventing diseases. Among these, the electrocardiogram (ECG or EKG) system is essential for assessing cardiac function. The ECG measures the cardiac cycle by capturing and recording the electrical activity of the heart during its contraction and relaxation phases.

Vital sign monitoring systems are susceptible to various types of noise, which can distort recorded signals and compromise accuracy. Common noise sources include baseline wander, electromyographic interference, motion artifacts, and electrode contact noise. Each noise type requires specific filtering or preprocessing techniques to enhance signal quality and reliability.

When an ECG lead or cable becomes disconnected, the effects vary depending on the context. Typically, the input circuit connects to a high impedance "pull-up" or "pull-down" resistor, causing the signal to saturate either high or low. In some cases, the remaining leads may still record an ECG, but the disconnected lead's high impedance to ground makes it particularly vulnerable to capacitively coupled noise, such as interference from mains power or high-frequency sources. This noise can contaminate the right leg drive (RLD) feedback mechanism, potentially affecting other leads.

Moreover, certain ECG leads rely on multiple electrodes to calculate and amplify accurate signals. For instance, if a limb lead is disconnected, the cable may pick up noise that weakens the overall ECG signal. The morphology of the ECG waveform may also change due to the loss of a recording site. Additionally, noise from the disconnected lead may propagate to other leads because of their linear interdependence, further degrading signal quality.

This issue is compounded by the challenge of distinguishing whether detected anomalies are genuine or caused by system-related noise. Misinterpretation of noisy signals can lead to inaccurate diagnoses or unnecessary interventions.

There is a clear need for advanced vital sign monitoring systems capable of detecting and mitigating noise to ensure the integrity of recorded signals. Such systems would improve diagnostic accuracy and reduce the risk of errors caused by noise contamination.

### Summary of the invention

To achieve at least one of the above objects and also other objects that will be evident from the following description, a system having the features defined in claim 1 is provided according to the present invention. Preferred embodiments will be evident from the dependent claims. The invention is defined by the appended claims only.

More specifically, there is provided according to a first aspect of the present invention a vital sign monitoring system. The system comprises at least two vital sign sensors, and a data processing unit.

The at least two vital sign sensors are configured to measure a same first vital sign parameter. The data processing unit is configured to:
- receive a data pertaining to said vital sign parameter from each one of said at least two vital sign sensors;
- analyze said data based on a criterion, and generate an output based on said analysis.

Within the context of the present application the term "vital sign monitoring system" is to be understood as a system used to monitor and track vital health parameters (such as heart rate, blood pressure, temperature, etc.) in real time to assess an individual's health status. This system often includes devices like ECG (electrocardiogram) monitors, which are configured to specifically measure the electrical activity of the heart to detect abnormalities in heart function.

Within the context of the present application the term "vital sign sensor" is to be understood as a device used to detect and measure specific vital sign parameters (e.g., heart rate, respiratory rate, temperature). This may include ECG sensors, which measure the electrical signals of the heart to monitor its rhythm and detect irregularities, such as for example arrhythmias.

Within the context of the present application the term "vital sign parameter" is intended to be understood as a measurable characteristic of a person's health, such as for example a heart rate, blood pressure, respiratory rate, or temperature, which is tracked to monitor health. This may include parameters measured by ECG, such as heart rate or heart rhythm.

Within the context of the present application the term "data processing unit" is to be understood as a component of a system responsible for processing the raw data collected by sensors, performing analysis based on the criteria, and generating output.

Analyses of criteria may for example be majority vote, excluding invalid measurements, criteria based on machine learning.

Generated output may for example be Selection or mean value of a sensor measure not deemed as invalid, or Time for a QRS event.

The data processing unit may be any data processing unit that may be able to analyze data related to vital sign parameters. The processing unit may for instance be implemented by a general-purpose processing unit, e.g., a central processing unit (CPU), which may execute instructions of one or more computer programs in order to implement functionality of the processor. The processor may also or alternatively be implemented as firmware arranged e.g., in an embedded system, or as a specifically designed processing unit, such as an Application-Specific Integrated Circuit (ASIC) or a Field-Programmable Gate Array (FPGA). It should be realized that the processing unit may be implemented as a combination of hardware and software components.

The system provides improved monitoring of vital systems. Patient safety may be improved since the system improves the accuracy of the data output pertaining to the vital sign that is monitored.

It is important and advantageous to have an improved system for monitoring vital sign parameters as this may improve the patient health and prevent avoidable emergencies. For example, detecting changes in a heart rate can indicate underlying issues such as arrhythmias, heart attacks, or other cardiovascular problems. Early detection of such patterns can lead to timely intervention.

Further the system can assess a condition to detect and identify the severity of such condition. As an example, a very high or very low heart rate can signal distress or deterioration.

Moreover, monitoring a vital sign parameter can improve evaluation of a patient's response to a treatment.

By proper monitoring with the provided system, potential problems can be caught at early stages, and thereby prevent avoidable complications.

The system may be used for heart monitoring, by continuously or periodically checking and recording the heart's activity to assess its function and detect any abnormalities.

The at least two vital signs sensors and the data processing unit may continuously monitor and analyze the output.

The term "output" is intended to refer to a result or a product that comes from the data processing unit after the analysis has been performed on the received data. For example, the output may be a vital sign parameter value that has resulted from the analysis. In other examples, the output may be an alarm or alert if the analysis shows an irregularity that may harm a patient that is monitored.

The output may be a QRS-complex, systolic blood pressure, diastolic blood pressure, ST segment measurement, QT interval measurement, the vital sign parameter value, a consolidated measurement, a time value or a real-time value of the vital sign parameter. It is to be noted that this is not an exhaustive list of possible outputs generated by the data processing unit. The generated output may be any combination of the listed examples, and/or in combination of further suitable outputs.

Within the context of the present application the term "QRS complex" is intended to be understood as referred to an ECG, where the QRS complex represents the electrical signals that trigger the contraction of the heart's ventricles.

Within the context of the present application the term "real-time value" is intended to be understood as the current measurement of a vital sign parameter at a specific moment, typically updated continuously to reflect ongoing changes in health status.

Within the context of the present application the term "consolidated measurement" is intended to be understood as a combined measurement from multiple vital sign sensors to provide a reliable and accurate measurement, preferably by first removing an invalid measurement.

Within the context of the present application the term "invalid measurement" is intended to be understood as a measurement that is incorrect or unreliable. This may be due to movement artifact, EMG artifact, disconnected sensors, noise, or other external factors that compromise accuracy.

Within the context of the present application the term "parameter value" is intended to be understood as a specific numerical measurement of a vital sign, such as a blood pressure reading or heart rate value, recorded by a sensor.

Within the context of the present application the term "time value" is intended to be understood as timestamp associated with a particular measurement or data point, indicating when it was recorded or taken. The time value may be a comparative time value between at least two time points. The time value may be more than one time points. The time value may be a specific time point.

The analysis of the data processing unit may generate an output that is within a safe range. The safe range is a determined range for a corresponding value which is considered a safe value. A safe value does not indicate danger to the patient and is a value that may be monitored.

When the output is within the safe range, no anomaly that may indicate a problem has been detected.

When an output or a data value is beyond the safe range, the value or output is identified as a non-safe value. Upon identifying a non-safe value, the output may be an alarm to alert a user of an anomaly that may need to be further investigated.

Generally, more than one vital sign parameter may be measured by each respective sensor and the measurements may be combined to provide output data for monitoring and/or diagnosing the patient.

However, it has been realized that such a system is susceptible to various types of noise, which can distort recorded signals and compromise accuracy of the output data. To provide a vital sign monitoring system which is resistant to such noise, it has been shown to be advantageous to have a system comprising at least two vital sign sensors configured to measure a same first vital sign parameter.

The data processing unit is configured to receive and analyze data pertaining to the first vital sign parameter from each one of said at least two vital sign sensors and generate an output based on the measurements of the same first vital sign parameter.

The data pertaining to said vital sign parameter may refer to a specific measurement and/or information collected about the vital sign.

The data processing unit is configured to analyze the vital sign parameter based on a criterion. In some examples, the criterion is a set of criteria. Depending on the criteria the data processing unit may be configured to disregard the measurements from one or more sensors if the first vital sign parameter is e.g., determined to be invalid. This provides the advantage of being able to disregard sensors not providing valid data which is needed in order to improve and achieve patient safety.

The term "criterion" is intended to refer to a standard, rules, and/or conditions used to evaluate or make decisions about the received data. It refers the specific guidelines or benchmarks that determine how the data is assessed or interpreted. The criterion is determined in order to generate a desired output. For example, if the system is configured to monitor a heart rate, the criterion may be related to patterns of heart rates, which may be used to detect an anomaly.

In some examples, the at least two vital sign sensors may be independent wireless self-contained sensors. Using wireless sensors remove the need for wired sensor which may facilitate the use of the system. The system may be easy to use for a patient in movement.

Within the context of the present application the term "independent sensor" is intended to be understood as sensors that operate separately from each other without dependencies.

Within the context of the present application the term "wireless self-contained sensors" is to be understood as sensors that do not require external wiring or connections to function, capable of transmitting data wirelessly to a processing unit or monitoring system.

Within the context of the present application the term "independent ECG sensors" is intended to be understood as ECG sensors that are placed in different locations on the body that can operate totally independent.

Each independent sensor may be a self-contained sensor operating independent of each of the other sensors. Thereby, there is no dependency on all sensors to obtain an accurate data output from the system and the sensors are less likely effected by the same source of interference. Hence, a system is provided which may improve patient safety.

In some examples the at least two vital sign sensors may be independent ECG sensors.

In some examples, a second vital sign sensor may be configured to measure the first vital sign parameter from a location different than a first vital sign sensor. Measuring at a different position is one manner of making the sensors independent as it may, as beforementioned, be less likely effected by same source of interference. It should be noted that more than two sensors may be configured to measure the first vital sign parameter, each sensor at a different location.

Within the context of the present application the term "a location different" is intended to refer to vital sign sensors are placed at different locations on the body, e.g. left arm (LA) and right arm (RA).

As an example, the at least two sensors may be placed at a respective position of the patient being e.g., right side of the chest, left side of the chest, right arm, left arm, right leg, left leg, a finger, etc.

By measuring the same vital sign at different locations may achieve "Spatial Redundancy" which may reduce localized anomalies from affecting the measurements. Such localized anomalies may for example be movement artifacts or radio interference.

In some examples, the data processing unit is configured to generate a consolidated measurement of said data received from each respective sensor.

The consolidated measurement may be a single value or a set of valued that represent the combines or aggregated results from the at least two sensors providing individual measurements of the vital sign parameter. The consolidated measurement may simplify complex data sets, thereby making it easier to analyze and draw meaningful conclusions form the generated output.

In some examples the consolidated measurement is a consolidated real-time value of the vital sign parameter.

The system may be configured to continuously output the consolidated real-time value of the vital sign parameter.

An advantage of configuring the data processing unit to generate a consolidated measurement is that the output may be based on the most reliable data sources. This allows sources affected by noise or artifacts to be effectively excluded from the consolidated measurement, even in the presence of transient interferences. As the vital sign sensors of the system measures the same vital sign parameter, the generated output may be more accurate compared to if a consolidated measurement is generated based on different vital sign parameters.

In some examples the data processing unit is configured to detect a QRS complex.

In some examples, the data processing unit is configured to generate a parameter value of the vital sign parameter corresponding to a time value.

The time value may be a specific time point value. The time value may be a comparative time value between at least two time points. The time value may be more than one time points.

The parameter value may be a comparative parameter value. The parameter value may be more than one parameter value.

In some examples the parameter value may be a systolic blood pressure value. In some examples the parameter value may be a diastolic blood pressure value. In some examples the parameter value may be a ST measurement. In some examples the parameter value may be a QT measurement.

In some examples, the data processing unit is configured to generate a time value as the output. The time value may be a specific time point value. The time value may be a comparative time value between at least two time points. The time value may be more than one time points.

In some examples the at least two sensors are configured to be synchronized to measure the first vital sign parameter at the same time. This is an advantage as it will facilitate the accuracy of detecting of anomalies. If the at least two sensors are synchronized the greater chance is that the measurements correspond to each other, thus reducing the noise if the measurements are compared or consolidated.

Within the context of the present application the term "synchronized to measure" is intended to be understood as the coordination of multiple devices or sensors to record measurements at the same time or in a coordinated manner for accurate comparison or analysis. In some examples the set of criteria is a set of predefined criteria.

Within the context of the present application the term "predefined criteria" may be understood as specific conditions or thresholds that have been set or defined.

It should be noted that the processing unit may be configured to run a sequence of analyses according to a defined sequence to generate the output. As an example, a first analysis may be based on a first criterion and thereafter a second analysis may be performed based on a second criterion, the process data unit may be configured to generate a separate output for each analysis or generate a consolidated measurement.

The set of criteria may comprise one or more criterion.

The data processing unit may be configured to identify an invalid measurement and/or an invalid source and remove said invalid measurement and/or invalid source from the analysis. In some examples an invalid source may be identified as a sensor providing an invalid measurement.

The invalid source is identified through a technical malfunction, i.e a technical disqualification.

The invalid source determined by a technical disqualification may use a technical signal to disqualify a measurement, thereby rendering the measurement disqualified. The technical signal may be a signal indicating whether the sensor is connected to the patient or not. Another technical signal may be a signal indicating that data is lost during the transmission between the measuring point, i.e., the sensor, and the analyzing point, i.e., the data processing unit. The data may be lost due to e.g., radio interference.

The invalid measurement is identified if the data received by the data processing unit is outside of a determined allowable range. The determined allowable range is intended to refer to a set of values within which a particular measurement or parameter is considered acceptable or not. The allowable range of the vital sign may be patient specific or be based on an expected range. The patient specific allowable range may be determined based on measurements of a vital sign performed during close observations, preferably with vital sign sensors connected by cables to the data processing unit.

The determined allowable range may indicate that the value is so far from what is expected that the value should be classified as an invalid measurement, thus not an acceptable measurement.

In some examples, the data processing unit may be configured to identify a same value received from more than 50% of the sensors and generate the output based on said same value.

In some examples, the data processing unit may be configured to identify a same value received from more than a threshold number of the sensors and generate the output based on said same value.

The threshold number may vary depending on how many sensors are present in the system. The threshold number may be dynamic as a sensor may be deemed invalid. The threshold number may for example be two in a system comprising three sensors.

The term "same value" intends to refer to a value being within a predefined allowable difference from one another. Meaning the same value must not be identical as a variation may be allowed to identify the measurements as same value. A same value may be identical readings or measurements taken by different sensors or devices, indicating consistency or agreement in the data. Note that a given difference in absolute value or time is typically accepted for the values to be considered same.

In some examples the criteria may be represented by a machine-learned model and the data processing unit may be configured to have access to the machine learned model for generating the output. The machine-learned model may be a system that uses algorithms based on previous data and learning to predict, classify, or output results autonomously, without direct human intervention.

According to a second aspect of the inventive concept, a method for monitoring a vital sign parameter.
measuring, by at least two vital sign sensors, a same first vital sign parameter,
receiving, by a data process unit, data pertaining to said vital sign parameter from each one of said at least two vital sign sensors,
analyzing, by the data processing unit, said data based on a criterion, and
generating an output based on said analyzed data.

The advantages of the various features which have been discussed above with reference to the system are equally applicable with reference to the method. Also, the various variants discussed with reference to the system are equally applicable to the method.

Generally, all terms used in the claims are to be interpreted according to their ordinary meaning in the technical field, unless explicitly defined otherwise herein. All references to "a/an/the [element, device, component, means, step, etc]" are to be interpreted openly as referring to at least one instance of said element, device, component, means, step, etc., unless explicitly stated otherwise. The steps of any method disclosed herein do not have to be performed in the exact order disclosed, unless explicitly stated.

### Brief description of the drawings

The above, as well as additional objects, features and advantages of the present invention, will be better understood through the following illustrative and nonlimiting detailed description of preferred embodiments of the present invention, with reference to the appended drawings, where the same reference numerals will be used for similar elements, wherein:
Figure 1 discloses a schematic perspective view of a vital sign monitoring system.
Figure 2a is a flow chart illustrating a method according to the present inventive concept.
Figure 2b is a flow chart illustrating an alternative method according to the present inventive concept.

### Description of embodiments

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled person.

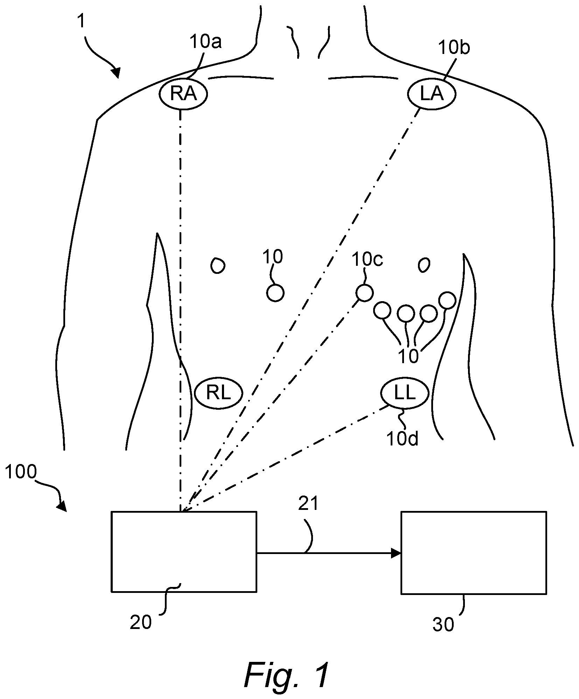

With reference to Fig.1, a vital sign monitoring system 100 is shown. The system 100 comprises four vital sign sensors 10a-d which are configured to measure a same first vital sign parameter. The first vital sign parameter may be a pulse rate or a hear rate, or a blood pressure. The first vital sign parameter that is measured, is the same vital sign parameter for each one of the sensors. Thus, each sensor is configured to measure the same vital sign parameter.

The system 100 requires at least two vital sign sensors. Hence the system 100 is not limited to the number of sensors depicted in the Figures. As an example, two, three, five, six, or more sensors may be used. Each sensor may be configured to measure the first vital sign parameter at different locations.

Each vital sign sensor 10a-d is placed at a respective location of the body of the patient 1, note that the illustrated placements are only illustrative and does not disclose preferred placements.

The Right arm (RA), Left arm (LA), Left leg (LL), and V2 may be particularly interesting locations to use when monitoring ECG. The Right leg (RL) may often be a grounding.

Measuring vital signs at a different position is one manner of making the sensors 10a-d independent as they may be less likely effected by same source of interference. Such interference may for example be baseline wander, electromyographic artefacts, motion artifacts, and high electrode impedance, EMC interference.

The vital sign sensors 10a-10d are preferably independent self-contained sensors. An independent self-contained sensor may operate independently without a need for external power or additional components to function. The independent self-contained sensor is designed to be compact and include necessary elements within the sensor.

The vital sign sensors 10a-d as shown in Fig. 1, may be independent wireless self-contained sensors configured to wirelessly transfer the respective measured first vital sign parameter to a data processing unit 20.

In other examples, the measurements made by the sensors may be transferred by wires to the data processing unit from the vital sign sensors.

The data processing unit 20 is configured to receive the data pertaining to the vital sign parameter from each one of the at least two vital sign sensors.

In an example, where the vital sign to be monitored is a heart rate, an example of data pertaining to the vital sign may be heart beats per minute.

The data processing unit 20 is configured to perform an analysis on the received data. The received data is based on a criterion. After and/or during the analysis the data processing unit 20 generates an output based on the analysis.

The vital signs sensors 10a-d and the data processing unit 20 may continuously monitor and analyze the first vital sign parameter. The output may be one out of QRS-complex, systolic blood pressure, diastolic blood pressure, ST segment measurement, QT interval measurement, or any other desired output to monitor.

As shown in Fig. 1, the data processing unit 20 may send the output 21 to an interface or a display unit 30. The interface or display unit 30 may configured to display the output 21 to a user. The user may thus control the system and the measurements from the interface unit 30. Thereby improved monitoring may be achieved by allowing for example a doctor to continuously observe and follow the data output from the vital sign monitoring system. The user interface may be interactive for the user to control the monitoring system.

In some examples the criterion may be a predefined criterion. In should be noted that the criterion may be a set of criteria.

In some examples, the system may comprise a storing unit configured to store the predefined criterion. In such examples, the data processing unit 20 may be configured to access the storing unit to analyze the data against the predefined criterion.

The criterion may be predetermined in order to generate a desired output from the analysis. For example, if the system is configured to monitor a heart rate, the criterion may be related to patterns of heart rates, which may be used to detect an anomaly. The criterion is generally set to determine how the data received by the data processing unit should be assessed and analyzed.

The data processing unit may be configured to run a predefined algorithm on the received data to generate the output 21. Such an algorithm may comprise one or more analyses.

The data processing unit may be configured to perform a technical disqualification analysis. In the technical disqualification, the data received from the sensors are analyzed to determine if there is a value that is not accepted, or not in the allowable range. The analysis may comprise analyzing the technical signal to disqualify a measurement from a sensor and thereby identifying it as an invalid source, before it is included further in the analysis. The technical signal may be a signal indicating whether the sensor is connected to the patient or not. Another signal may be a signal indicating that data is lost during the transmission between the measuring point, i.e., the sensor, and the analyzing point, i.e., the data processing unit, due to e.g., radio interference.

When a sensor is disqualified, an alarm may be outputted by the data processing unit. Thereby a user may investigate the sensor and correct any technical issues.

The data processing unit may be configured to identify an invalid source through a source output consistency analysis. As an example, the data processing unit may compare a sensor's recent outputs with a number of prior outputs from the processing unit. If the sensor's outputs deviate beyond a predefined threshold, e.g., exceeding a 30% error rate, the source may be classified as invalid. The invalid source sensor may remain as invalid until its performance improves, e.g., maintaining an accuracy above a 70% threshold. After the performance has reached an acceptable level the sensor may be identified as a valid source, and measurements form the sensor may be included in a further analysis performed by the data processing unit.

It is to be understood that the number of prior last readings may be at least 5, preferably at least 10, more preferably at least 15. However, it is to be understood that it may be any desired number.

It is to be understood that the error rate may be different from 30%. The error rate may be a value in the range of 10%-90%, preferably 20%-60%, more preferably 30-40%.

It is to be understood that the accuracy may be different from 70%. The accuracy rate may be at least 50%, preferably at least 60%, more preferably at least 70%.

Another method for invalidating sources may be based on the detection of different artifacts such as EMG detection, movement artifacts, or high noise levels.

The data processing unit may be configured to identify an invalid measurement. The data processing unit 20 may analyze the received data based on the determined allowable range for the respective vital sign parameter. If the received data value is within the allowable range, the measurement is identified as valid. When the received data value is beyond the allowable range, the measurement is identified as invalid. When a measurement is identified as invalid, the data processing unit will remove the invalid measurement from the analysis.

In some examples, the data processing unit may be configured to compare the received data from each vital sign sensor. In such examples, the processing unit may be configured to disregard received data that deviates from the other data by identifying the received data as an invalid measurement.

When comparing the received data, the data processing unit 20 may determine a difference between the received values. When the difference is within a threshold, or a predefined allowable difference, the output may be based on all the received data. Further, if the comparison shows that there is little to no difference between the received data, no invalid measurement is identified.

If a difference is detected that is above the predefined allowable difference of below the threshold, the measurement may be identified as an invalid measurement and removed from the analysis and thereby removed from the output.

By comparing the received data and received values, the data processing unit may determine if a majority of the vital sign sensors provide the same measurement value of the vital sign parameter, there by provide the output as a majority vote. Hence, if a majority of the vital sign sensors provide data of the same value within a predefined allowable difference, these data may be used to generate the output. For example, the majority vote result may generate a mean value of the vital sign parameter value, as a consolidated measurement.

The term "same value" intends to refer to a value being within a predefined allowable difference.

The vital sign monitoring system may analyze the measured vital sign parameter from each one of said at least two vital sign sensors and compare the data with criteria and generate a separate output for each sensor and/or generate a consolidated measurement output based on all sensors. The combined output may be a mean of the data provided by each sensor.

In some examples the analysis may be based on a majority vote with a dynamic threshold. The required number of sources having the same measured value may be changed depending on whether one or more sensors are disqualified-rendered invalid sources. For a majority vote, the data processing unit is configured to identify the same value from more than a threshold number of the sensors. Thereafter, the output may be based on said same value.

As an example, where the vital sign monitoring system comprises six sensors. One of the six sensors may be disconnected and determined to be disqualified due to delivering invalid measurement. The majority threshold- number meaning how many sensor values are needed for a majority vote changes from four to three sources.

Furthermore, the criteria may comprise the determined safe range for the vital sign parameter. The safe range may be of the specific patient's vital sign or the allowable range of the expected vital sign. Hence, the data processing unit may analyze the received data based on the safe range to detect anomalies. The determined safe range may provide indication of anomalies, or issues that are beneficial to detect in order to avoid emergencies. If the data processing unit detects a value or if the analysis generates a value that is beyond the safe range, but not determined as not acceptable by the allowable range, the data processing unit is configured to generate an alarm. The alarm may be displayed on the interface or display unit 30. The interface or display unit 30 may comprise an audio unit and/or light unit configured to be activated when receiving an alarm from the data processing unit. Thereby alerting any user operating the interface or display unit 30.

The system may comprise a recording unit for recording and storing measurements and the generated output.

In some examples, the system may be subjected to a synchronization method for synchronizing the sensors. Meaning that the sensors may be synchronized to ensure that they record measurements at the same time.

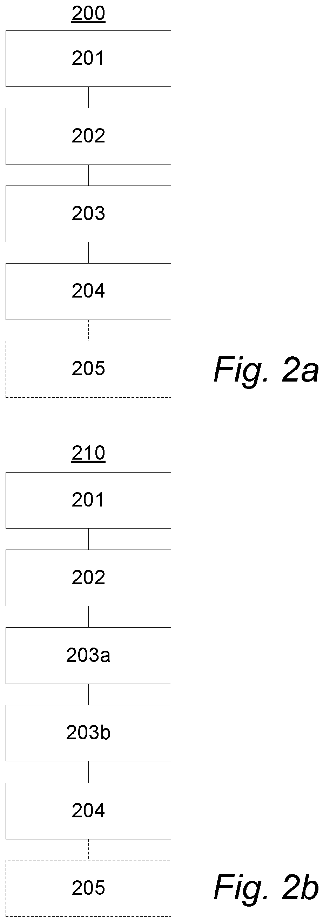

Turning to Fig. 2a, a method 200 according to an aspect of the present inventive concept will now be described. At a first step 201, the vital sign sensors of the vital sign monitoring system 100 measure a first vital sign parameter. The first vital sign parameter may be one out of pulse rate, heart rate, respiratory rate, blood pressure, body temperature or any other suitable vital sign parameter.

At the measuring step 201, the at least two sensors 10a-d of the vital sign monitoring system 100 may be synchronized to measure the first vital sign parameter at the same time. It should be noted that the vital sign sensors10a-d are configured to measure the vital sign parameter at the same time, however the timing for taking the measurements may become unsynchronized after some time of measuring. Therefore, it is advantageous to incorporate a synchronization activity to ensure accurate measurements.

The data processing unit 20 receives the measured data at step 202. The processing unit may analyze the received data at step 203 based on a criterion or a set of criteria. One such criterion may be to analyze whether or not the measurement from each sensor falls within the determined allowable range. The allowable range of vital sign may be patient specific or be based on an expected range. The patient specific allowable range may be based on earlier measurements.

In step 204 the data processing unit 20 generates an output 21 based on the analyzed data. The output 21 unit is based e.g. on the remaining valid measurements and/or on a mean value of measurements being considered to be of the same value, or a consolidated value of all the measurements.

Optionally, the data process unit 20 transmits 205 the output to an interface or display unit.

With reference to Fig. 2b, an alternative method 210 is shown. As described in relation to method 200, the vital sign sensors of the vital sign monitoring system 100 measure 201 a first vital sign parameter. The data processing unit 20 receives the measured data at step 202 and the processing unit 20 analyses the received data at step 203 based on a set of criteria. In Fig. 2b, there are two analyzing steps 203a, 203b. The method may comprise more than two analyzing steps. For example, the method may comprise a sequence of analyses performed by the data processing unit 20.

A first analysis 203a may be based on a criterion to identify and remove invalid source or measurement, either by using a technical disqualification signal or for example through source output consistency analysis. When an invalid measurement has been removed, the data processing unit 20 may perform a second analysis 203b based on a second criterion. The second criterion may be based on a majority vote with a static or dynamic threshold. At this second analyzing step 203b, the data processing unit 20 analyses the data provided by the remaining valid measurements and determines whether a majority of the valid sources has provided the same value. Thereafter the output is generated based on the same value analysis which is optionally transmitted 205 to an interface or display unit 30.

It is to be understood that the different criteria presented herein could be combined in other sequences. The method may comprise more than two steps of analysis in order to generate the output 21.

In some examples, the criteria may be represented by a machine-learned model. In such examples, the data processing unit 20 may be configured to have access to the machine learned model for generating the output.

In some examples, the system may be calibrated before the use on a specific patient. The system may be connected via ECG cables to retrieve the any of standard ECG lead (I, II, III, aVR, aVL, aVF, V1,...,V6). The parameters to derive the standard ECG leads from the locally recorded ECG may then be calculated and adapted to the individual patient resulting in the generation of derived standard leads using ECG patches. By connecting the system to for example a computer, the system may generate a model for the unique heart. The calibration of the system may take a minute or longer depending on the readings made by the sensors and the system. The machine learning model may be updated and trained by the data collected during the calibration, when the sensors are connected to establish the hearts ECG pattern both for the standard ECG leads and for the local ECG recordings.

The system can learn what is considered a normal heart rhythm for an individual heart. It can also determine the typical ECG curve form for that specific patient and the corresponding ECG sensor. A parameter table may be created corresponding to the normal heart rhythm of the patient or the normal ECG curve form. The parameter table may be created from the calibration of the system or continuously. When enough data has been provided, the system is applied to monitor the patient. The data processing unit may use the parameter table when analyzing the data received from the at least two sensors. Thereby, any irregularities may be detected.

All measurements and generated output based on the analyses of the data pertaining to said measurement may be integrated into the machine learning model. Thereby the machine learning model may be continuously updated. Moreover, the accuracy of the machine learning model may be improved by historic data being entered into the model.

The model may use a recursive neural network. The model may use a convolutional neural network.

It will be appreciated that the present invention is not limited to the embodiments shown. Several modifications and variations are thus conceivable within the scope of the invention which thus is exclusively defined by the appended claims.

## Claims

1. A vital sign monitoring system, comprising:
at least two vital sign sensors being configured to measure a same first vital sign parameter,
a data processing unit (20) configured to:
- receive data pertaining to said vital sign parameter from each one of said at least two vital sign sensors;
- analyze said data based on a criterion and generate an output (21) based on said analysis.

2. The vital sign monitor system (100) of claim 1, wherein the at least two vital sign sensors are independent wireless self-contained sensors.

3. The vital sign monitor system (100) of claim 1 or 2, wherein the at least two vital sign sensors are independent ECG sensors.

4. The vital sign monitor system (100) of any one of the preceding claims, wherein a second vital sign sensor (10) is configured to measure the first vital sign parameter from a location different than a first vital sign sensor.

5. The vital sign monitor system (100) of claim 1, wherein the data processing unit (20) is configured to generate a consolidated measurement of said data received from each respective sensor based on the analyze.

6. The vital sign monitor system (100) of claim 5, wherein the consolidated measurement is a consolidated real-time value of the vital sign parameter.

7. The vital sign monitor system (100) of any one of the preceding claims, wherein the data processing unit (20) is configured to detect a QRS complex.

8. The vital sign monitor system (100) of any one of the preceding claims, wherein the data processing unit (20) is configured to generate a parameter value of the vital sign parameter corresponding to a time value.

9. The vital sign monitor system (100) of any one of the preceding claims, wherein the data processing unit (20) is configured to generate a time value as the output.

10. The vital sign monitor system (100) of any one of the preceding claims, wherein the at least two vital sign sensors are configured to be synchronized to measure the first vital sign parameter at the same time.

11. The vital sign monitor system (100) of any one of the preceding claims, wherein the set of criteria is a set of predefined criteria.

12. The vital sign monitor system (100) of any one of the preceding claims, wherein the data processing unit (20) is configured to identify a same value received from more than a threshold number of the sensors and generate an output based on said same value.

13. The vital sign monitor system (100) of any one of the preceding claims, wherein the data processing unit (20) is configured to identify an invalid measurement and remove said invalid measurement from the analysis.

14. The vital sign monitor system (100) of any one of the preceding claims, wherein the criteria may be represented by a machine-learned model and wherein the data processing unit (20) is configured to have access to the machine learned model for generating the output.

15. A method for monitoring vital signs, the method comprising:
measuring (201), by at least two vital sign sensors, a same first vital sign parameter,
receiving (202), by a data processing unit (20), data pertaining to said vital sign parameter from each one of said at least two vital sign sensors,
analyzing (203), by the data processing unit, said data based on a set of criteria, and
generating (204) an output (21) based on said analyzed data.

## Amended claims

### Amended claims in accordance with Rule 137(2) EPC.

1. A vital sign monitoring system, comprising:
at least two self-contained vital sign sensors being configured to measure a same first vital sign parameter and operating independent of each of the other self-contained vital sign sensor,
a data processing unit (20) configured to:
- receive data pertaining to said same vital sign parameter from each one of said at least two self-contained vital sign sensors;
- analyze said data based on a criterion to identify and remove an invalid measurement, and generate an output (21) based on said analysis.

2. The vital sign monitor system (100) of claim 1, wherein the at least two vital sign sensors are independent ECG sensors.

3. The vital sign monitor system (100) of any one of the preceding claims, wherein a second vital sign sensor (10) is configured to measure the first vital sign parameter from a location different than a first vital sign sensor.

4. The vital sign monitor system (100) of claim 1, wherein the data processing unit (20) is configured to generate a consolidated measurement of said data received from each respective sensor based on the analyze.

5. The vital sign monitor system (100) of claim 4, wherein the consolidated measurement is a consolidated real-time value of the vital sign parameter.

6. The vital sign monitor system (100) of any one of the preceding claims, wherein the data processing unit (20) is configured to detect a QRS complex.

7. The vital sign monitor system (100) of any one of the preceding claims, wherein the data processing unit (20) is configured to generate a parameter value of the vital sign parameter corresponding to a time value.

8. The vital sign monitor system (100) of any one of the preceding claims, wherein the data processing unit (20) is configured to generate a time value as the output.

9. The vital sign monitor system (100) of any one of the preceding claims, wherein the at least two vital sign sensors are configured to be synchronized to measure the first vital sign parameter at the same time.

10. The vital sign monitor system (100) of any one of the preceding claims, wherein the set of criteria is a set of predefined criteria.

11. The vital sign monitor system (100) of any one of the preceding claims, wherein the data processing unit (20) is configured to identify a same value received from more than a threshold number of the sensors and generate an output based on said same value.

12. The vital sign monitor system (100) of any one of the preceding claims, wherein the criteria may be represented by a machine-learned model and wherein the data processing unit (20) is configured to have access to the machine learned model for generating the output.

13. A method for monitoring vital signs, the method comprising:
measuring (201), by at least two self-contained vital sign sensors, a same first vital sign parameter independent of each other,
receiving (202), by a data processing unit (20), data pertaining to said same vital sign parameter from each one of said at least two self-contained vital sign sensors,
analyzing (203), by the data processing unit, said data based on a set of criteria
identify and remove an invalid measurement, and
generating (204) an output (21) based on said analyzed data.
